# EUROPEAN PATENT SPECIFICATION

(11) **EP 2 729 102 B1**
(45) Date of publication and mention of the grant of the patent: **10.12.2014**
(21) Application number: 12743542.8
(22) Date of filing: 05.07.2012
(51) Int. Cl.: B05C 1/08, B05C 1/10, A61F 13/15, B05C 1/16, B05C 19/00, B05C 19/06

(54) **SHEET ARTICLE MANUFACTURING APPARATUS**
VORRICHTUNG ZUR HERSTELLUNG EINES FOLIENARTIKELS
APPAREIL DE FABRICATION D'ARTICLES DE FEUILLES

(30) Priority: 08.07.2011 JP 2011151577
(43) Date of publication of application: 14.05.2014
(73) Proprietor: Livedo Corporation, ShikokuChuo-shi Ehime 799-0122 (JP)
(72) Inventor: MARUHATA, Kazuya, Mima-gun Tokushima 779-4104 (JP)
(74) Representative: TBK
(86) International application number: PCT/JP2012/004386
(87) International publication number: WO 2013/008430

(56) References cited:
- EP-A1- 1 655 007
- EP-A2- 1 621 167
- EP-A2- 1 700 586
- JP-A- 2007 130 818

## Description

### Technical Field

The present invention relates to a sheet article manufacturing apparatus for manufacturing a sheet article for an absorbent article.

### Background Art

Absorbent sheets are conventionally manufactured by supplying a sheet member with particles of high-absorbent resin and placing another sheet member on the above sheet member to bond with each other. For example, in an apparatus of Japanese Patent Application Laid-Open No. 2005-59579 (Document 1), a temporary receiving roller and a transfer roller which are rotated in directions opposite to each other are provided. In the temporary receiving roller, rows of concave grooves arranged on its roller surface at a constant pitch are formed. Each concave groove receives high-absorbent resin particles and holds them in the form of layers. The particles are moved to a position right above a base sheet held on a roller surface of the transfer roller, and the particles are transferred onto a top surface of the base sheet on which hot melt adhesive is applied. In addition, the apparatus of Document 1 is provided with a pressure roller for bonding a cover sheet on the base sheet by pressure bonding. Immediately after the base sheet on which the high-absorbent resin particle layer has been transferred comes through a gap between the temporary receiving roller and the transfer roller, the base sheet is covered with the cover sheet.

Incidentally, in a case like the apparatus of Document 1, a plurality of concave portions each filled with particles of high-absorbent resin are arranged on an outer side surface of a cylinder part in a circumferential direction, a sheet conveying roller conveys a sheet member to the vicinity of the cylinder part while the cylinder part is rotated, and therefore the particles are supplied onto the sheet member. However, in the above case, particles ejected from the cylinder part actually collide with an outer side surface of the sheet conveying roller through the sheet member (i.e., the particles collide with the sheet member on the sheet conveying roller), and as a result, some particles are scattered around and wasted. Therefore, a technique of reducing scattering of particles ejected toward the sheet member is required.

### Summary of Invention

The present invention is intended for a sheet article manufacturing apparatus for manufacturing a sheet article for an absorbent article. It is an object of the present invention to reduce scattering of particles ejected toward a sheet member.

The sheet article manufacturing apparatus according to the present invention comprises: a cylinder part having an outer side surface which is generally cylindrical around a rotation axis along a horizontal direction, a plurality of holes each filled with particles of absorbent material or deodorant material being arranged on the outer side surface in a circumferential direction around the rotation axis, the outer side surface being rotated around the rotation axis in a predetermined rotation direction, the cylinder part ejecting the particles almost along a tangent line of the outer side surface at an ejection start position when each of the plurality of holes passes the ejection start position, the ejection start position being set at a position in a vicinity of a lowermost portion in a cross section of the outer side surface which is orthogonal to the rotation axis, the position lying posterior to the lowermost portion in the rotation direction; a sheet conveying roller which is located near the lowermost portion of the cylinder part, the sheet conveying roller having an outer side surface which is generally cylindrical around a central axis parallel to the rotation axis, an annular groove along a circumferential direction around the central axis being formed on the outer side surface so as to face holes of the cylinder part, the outer side surface being rotated around the central axis in a rotation direction opposite to the rotation direction of the cylinder part to convey a first sheet member along the outer side surface which is continuous sheet and cause the first sheet member to pass in the vicinity of the lowermost portion of the cylinder part; and a conveying and bonding part for placing, in the vicinity of the lowermost portion of the cylinder part, a second sheet member on the first sheet member which has been supplied with the particles to bond the second sheet member on the first sheet member, the second sheet member being continuous sheet; wherein the tangent line intersects with the outer side surface of the sheet conveying roller.

In the present invention, it is possible to reduce scattering of particles ejected toward the first sheet member and as a result, particles can be accurately fixed on a region of a sheet article for an absorbent article corresponding to the annular groove.

According to a preferred embodiment of the present invention, a first intersection point at which the tangent line intersects with the outer side surface of the sheet conveying roller lies posterior to a second intersection point in the rotation direction of the sheet conveying roller, at which a line segment connecting the rotation axis of the cylinder part and the central axis of the sheet conveying roller intersects with the outer side surface of the sheet conveying roller. Therefore, even if particles bounce from the first sheet member in the annular groove, scattering of particles can be suppressed by portions of the outer side surface of the cylinder part which are near the sheet conveying roller.

According to another preferred embodiment of the present invention, the conveying and bonding part comprises: another sheet conveying roller which is located anterior to the lowermost portion of the cylinder part with respect to a moving direction of the outer side surface at the lowermost portion, the another sheet conveying roller having an outer side surface which is generally cylindrical around a central axis parallel to the rotation axis, for conveying the second sheet member along the outer side surface to the vicinity of the lowermost portion to place the second sheet member on the first sheet member which has been supplied with the particles; and a sheet bonding part for bonding the second sheet member on the first sheet member; wherein the another sheet conveying roller has a groove or an absorber on the outer side surface, the groove extending along substantially the entire length of the outer side surface in a circumferential direction around the central axis and facing holes of the cylinder part, the absorber being configured to absorb impact on the particles which collide with the second sheet member on the outer side surface. As a result, it is possible to further reduce scattering of particles since impact on particles which collide with the second sheet member on the another sheet conveying roller is absorbed.

According to still another preferred embodiment of the present invention, the plurality of holes are formed on the outer side surface of the cylinder part with respect to each of a plurality of positions in an axial direction parallel to the rotation axis, and the annular groove is formed on the outer side surface of the sheet conveying roller with respect to each of the plurality of positions in the axial direction. This allows particles to be accurately fixed on a plurality of strip-like regions of a sheet article for an absorbent article.

These and other objects, features, aspects and advantages of the present invention will become more apparent from the following detailed description of the present invention when taken in conjunction with the accompanying drawings.

### Brief Description of Drawings

[fig.1]Fig. 1 is a view showing a structure of an absorbent sheet manufacturing apparatus.
[fig.2]Fig. 2 is a view showing an outer side surface of a cylinder part.
[fig.3]Fig. 3 is a cross-sectional view of a first sheet conveying roller.
[fig.4]Fig. 4 is a view showing a vicinity of a lowermost portion of the cylinder part.
[fig.5]Fig. 5 is a cross-sectional view of a second sheet conveying roller.
[fig.6]Fig. 6 is a cross-sectional view of a bonding roller.
[fig.7]Fig. 7 is an enlarged view showing the vicinity of the lowermost portion of the cylinder part.
[fig.8]Fig. 8 is a view showing an absorbent sheet.
[fig.9]Fig. 9 is a view showing a comparative example of absorbent sheet manufacturing apparatus.
[fig.10]Fig. 10 is a view showing another example of absorbent sheet manufacturing apparatus.
[fig.11]Fig. 11 is an enlarged view showing a vicinity of a lowermost portion of a cylinder part.
[fig.12]Fig. 12 is a view showing another example of particle supplying part.
[fig.13]Fig. 13 is a view showing an outer side surface of a cylinder part.
[fig.14]Fig. 14 is a view showing another example of second sheet conveying roller.

### Description of Embodiments

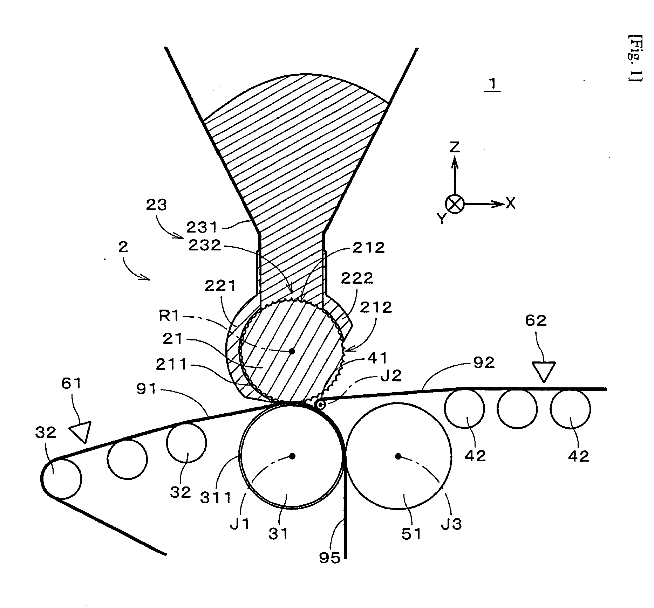

Fig. 1 is a view showing a structure of an absorbent sheet manufacturing apparatus 1 in accordance with a preferred embodiment of the present invention. The absorbent sheet manufacturing apparatus 1 is one sheet article manufacturing apparatus for manufacturing a sheet article for an absorbent article and manufactures absorbent sheets by sandwiching particles of high-absorbent resin such as SAP (Super Absorbent Polymer) between sheet members formed of nonwoven fabric or the like. The absorbent sheet is a sheet article used for an absorbent article such as a disposable diaper or absorbent pad for light incontinence.

The absorbent sheet manufacturing apparatus 1 has a cylinder part 21 having a generally columnar shape around (with its center lying on) a rotation axis R1 parallel to a predetermined axial direction (the Y direction in Fig. 1) along a horizontal direction, a first sheet conveying roller 31 having a generally columnar shape around a first central axis J1 parallel to the axial direction, a second sheet conveying roller 41 having a generally columnar shape around a second central axis J2 parallel to the axial direction, and a bonding roller 51 having a generally columnar shape around a third central axis J3 parallel to the axial direction. In Fig. 1, another horizontal direction orthogonal to the axial direction is shown as the X direction, and the vertical direction (i.e., direction of gravitational force) orthogonal to the X direction and the Y direction is shown as the Z direction.

The cylinder part 21, the second sheet conveying roller 41 and the bonding roller 51 are rotated in a counterclockwise direction in Fig. 1, and the first sheet conveying roller 31 is rotated in a clockwise direction in Fig. 1. The first sheet conveying roller 31 is located under (on the (-Z) side of) the cylinder part 21 and conveys a first sheet member 91 formed of nonwoven fabric or the like to cause the first sheet member 91 to pass under a lowermost portion (i.e., an outermost portion on the (-Z) side) of the cylinder part 21. The second sheet conveying roller 41 has a smaller diameter than those of the cylinder part 21 and the first sheet conveying roller 31, and is located in the vicinity of the lowermost portion of the cylinder part 21. The second sheet conveying roller 41 conveys a second sheet member 92 formed of nonwoven fabric or the like to the vicinity of the lowermost portion of the cylinder part 21. The bonding roller 51 is provided beside the first sheet conveying roller 31.

The cylinder part 21 has an outer side surface 211 which is a generally cylindrical surface around the rotation axis R1. A first cover part 221 which covers a portion of the outer side surface 211 and a second cover part 222 which covers another portion of the outer side surface 211 are provided around the cylinder part 21. In Fig. 1, the cylinder part 21, the first cover part 221, the second cover part 222 and an after-mentioned particle filling part 23 are shown as cross sections which are orthogonal to the rotation axis R1.

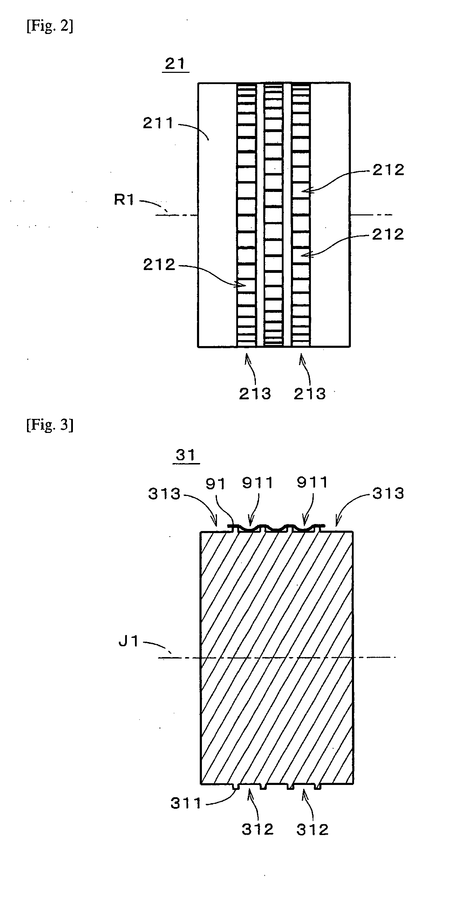

Fig. 2 is a view showing the outer side surface 211 of the cylinder part 21, and in Fig. 2, an appearance of the outer side surface 211 of the cylinder part 21 which is observed along a direction orthogonal to the rotation axis R1 is shown. In Fig. 2, the first and second cover parts 221, 222 are omitted. A plurality of concave portions 212 are arranged on (in) the outer side surface 211 of the cylinder part 21 in a circumferential direction around the rotation axis R1 with respect to each of a plurality of positions in the axial direction. When the plurality of concave portions 212 which are arranged in the circumferential direction at the same position in the axial direction are referred to as a concave portion row 213, three concave portion rows 213 are formed in the cylinder part 21 shown in Fig. 2. In Fig. 2, a shape of opening of each concave portion 212 which is a hole is generally rectangular, however the aperture may have a various shape (for example, generally circular shape).

As shown in Fig. 1, a particle filling part 23 storing particles of high-absorbent resin is provided above the cylinder part 21, and the particle filling part 23 fills the plurality of concave portions 212 of the cylinder part 21 with the particles. The particle filling part 23 has a particle tank 231 storing the particles, and a particle filling opening 232 facing the outer side surface 211 of the cylinder part 21 is provided under the particle tank 231. A not-shown level sensor is provided to the particle tank 231, and therefore particles are replenished into the particle tank 231 when the amount of particles stored in the particle tank 231 becomes equal to or less than a certain level.

The outer side surface 211 of the cylinder part 21 is rotated around the rotation axis R1 at a high speed in the counterclockwise direction in Fig. 1 by a not-shown motor, and particles are filled into each concave portion 212 passing the particle filling opening 232, by gravity. The first cover part 221 spreads from the particle filling part 23 toward the anterior side (downstream side) in the rotation direction of the cylinder part 21. Each concave portion 212 is closed (blocked) with the first cover part 221 after passing the particle filling part 23 until reaching the vicinity of the lowermost portion of the cylinder part 21. When each concave portion 212 passes the tip of the first cover part 221 (i.e., one end in the vicinity of the lowermost portion of the cylinder part 21), the particles in the concave portion 212 are ejected. As described later, particles ejected from the concave portion 212 are held on the first sheet member 91. In the following description, a position of the tip of the first cover part 221 in a cross section of the outer side surface 211 which is orthogonal to the rotation axis R1 is referred to as an "ejection start position". Details of ejection of particles from the cylinder part 21 will be discussed later.

The second cover part 222 spreads from the particle filling part 23 toward the posterior side (upstream side) in the rotation direction of the cylinder part 21. Each concave portion 212 which has ejected particles is closed with the second cover part 222 immediately before reaching the particle filling part 23. In the absorbent sheet manufacturing apparatus 1, the cylinder part 21, the first cover part 221, the second cover part 222 and the particle filling part 23 construct a particle supplying part 2 for supplying particles of high-absorbent resin onto the first sheet member 91.

Fig. 3 is a cross-sectional view of the first sheet conveying roller 31 and shows a cross section of the first sheet conveying roller 31 taken along a plane including the rotation axis R1 of the cylinder part 21 in Fig. 1 and the first central axis J1 of the first sheet conveying roller 31. The first sheet conveying roller 31 shown in Fig. 1 is located in the vicinity of the lowermost portion of the cylinder part 21. As shown in Fig. 3, the first sheet conveying roller 31 has an outer side surface 311 which is a generally cylindrical surface around the first central axis J1, and an annular groove 312 along a circumferential direction around the first central axis J1 is formed on (in) the outer side surface 311 with respect to each of the plurality of positions in the axial direction. The plurality of annular grooves 312 face the plurality of concave portion rows 213 of the cylinder part 21, respectively (that is, the annular grooves 312 are disposed at same positions in the axial direction as the positions of the concave portion rows 213). Annular cutout portions 313 are formed at both end portions in the axial direction on the outer side surface 311 of the first sheet conveying roller 31 in Fig. 3. The cutout portions 313 are not necessarily provided.

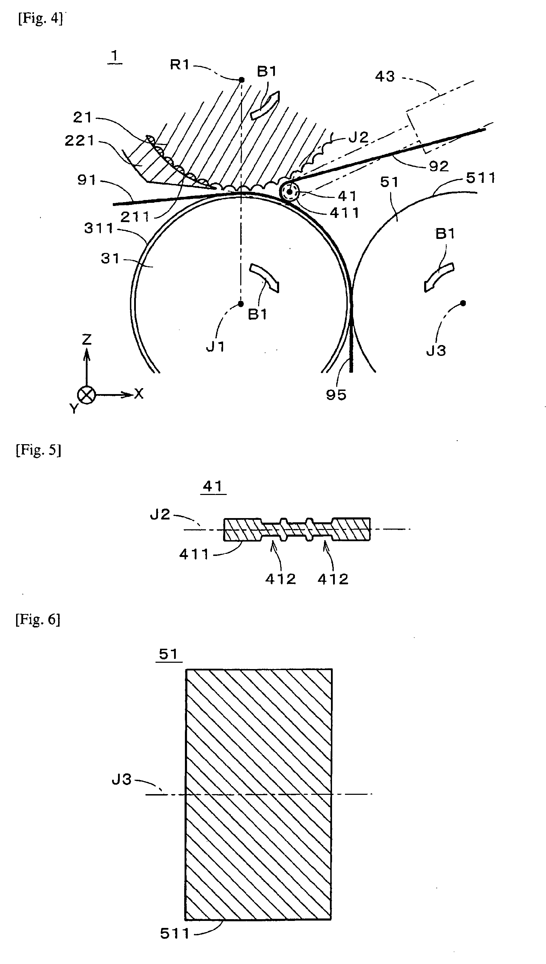

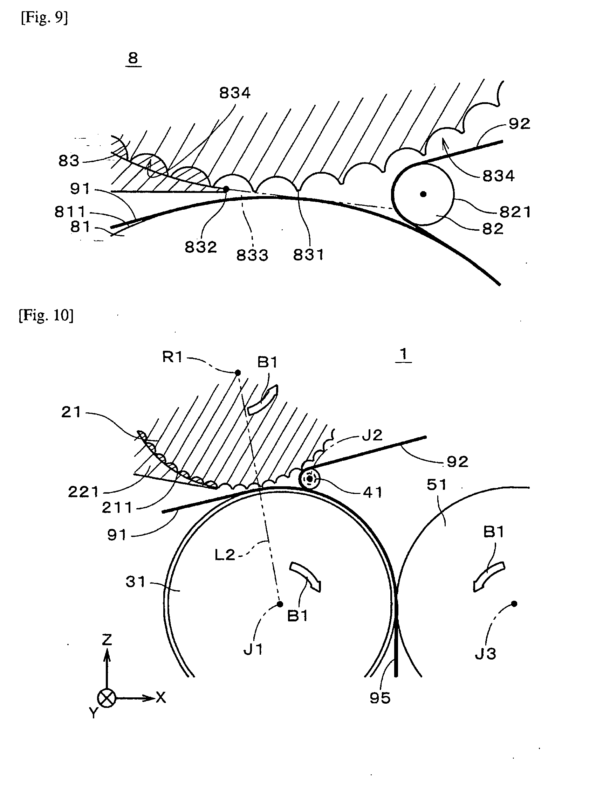

Fig. 4 is an enlarged view showing the vicinity of the lowermost portion of the cylinder part 21 in Fig. 1. In Fig. 4, the rotation directions of the cylinder part 21, the first sheet conveying roller 31 and the bonding roller 51 are shown by arrows denoted by a reference sign B1 (the same applies to after-mentioned Fig. 10). The outer side surface 311 of the first sheet conveying roller 31 is slightly away from the outer side surface 211 of the cylinder part 21, and the outer side surface 311 is rotated around the first central axis J1 in the rotation direction (in the clockwise direction in Fig. 4) opposite to the rotation direction of the cylinder part 21 as mentioned previously. The first sheet member 91 which is continuous sheet is led to the first sheet conveying roller 31 through a plurality of auxiliary rollers 32 (see Fig. 1), to be conveyed continuously along the outer side surface 311 of the first sheet conveying roller 31. At this time, the first sheet member 91 passes the vicinity of the lowermost portion of the cylinder part 21, and particles ejected from the cylinder part 21 are held on one main surface thereof. The movement speed of the outer side surface 211 at the lowermost portion of the cylinder part 21 is different from (or may be same as) the movement speed of the outer side surface 311 at the uppermost portion of the first sheet conveying roller 31.

Fig. 5 is a cross-sectional view of the second sheet conveying roller 41 and shows a cross section of the second sheet conveying roller 41 taken along a plane including the second central axis J2 of the second sheet conveying roller 41 in Fig. 4. With respect to a moving direction (the X direction in Fig. 1) of the outer side surface 211 of the cylinder part 21 at the lowermost portion, the second sheet conveying roller 41 shown in Fig. 4 lies anterior to the lowermost portion. As shown in Fig. 5, the second sheet conveying roller 41 has an outer side surface 411 which is a generally cylindrical surface around the second central axis J2, and an annular groove 412 along a circumferential direction around the second central axis J2 is formed on the outer side surface 411 with respect to each of the plurality of positions in the axial direction. The plurality of annular grooves 412 face the plurality of the concave portion rows 213 of the cylinder part 21 (and the plurality of annular grooves 312 of the first sheet conveying roller 31), respectively.

As mentioned previously, since the diameter of the second sheet conveying roller 41 in Fig. 4 is sufficiently-small in comparison with those of the cylinder part 21 and the first sheet conveying roller 31, the second sheet conveying roller 41 can be easily located in the vicinity of the lowermost portion of the cylinder part 21. In addition, the outer side surface 411 of the second sheet conveying roller 41 is rotated around the second central axis J2 in the counterclockwise direction in Fig. 1. The second sheet member 92 which is continuous sheet is led to the second sheet conveying roller 41 through a plurality of auxiliary rollers 42 (see Fig. 1), to be conveyed continuously along the outer side surface 411 of the second sheet conveying roller 41. Then, the second sheet member 92 is placed (stacked) on the first sheet member 91 which has passed under the lowermost portion of the cylinder part 21. The second sheet conveying roller 41 can be moved between the position shown in Fig. 4 and a position on the (+X) side of the cylinder part 21 by a forward/backward moving mechanism 43 shown by chain double-dashed lines in Fig. 4.

Fig. 6 is a cross-sectional view of the bonding roller 51 and shows a cross section of the bonding roller 51 taken along a plane including the third central axis J3 of the bonding roller 51 in Fig. 4. As shown in Fig. 6, the bonding roller 51 has an outer side surface 511 which is a cylindrical surface around the third central axis J3, the outer side surface 511 is a smooth surface. As shown in Fig. 4, the first sheet member 91 and the second sheet member 92 overlaid with each other are placed (sandwiched) between the outer side surface 311 of the first sheet conveying roller 31 and the outer side surface 511 of the bonding roller 51. The both (or one) of the first sheet conveying roller 31 and the bonding roller 51 are provided with heaters, and regions of the first sheet member 91 and the second sheet member 92 which come into contact with the outer side surface 311 of the first sheet conveying roller 31 (i.e., portions of the outer side surface except for the annular grooves 312 and the cutout portions 313) are heat-sealed with each other, so that the first sheet member 91 and the second sheet member 92 are bonded with each other.

As shown in Fig. 1, the absorbent sheet manufacturing apparatus 1 further has a first applying part 61 for applying adhesive (in the present embodiment, hot melt adhesive) onto the first sheet member 91 and a second applying part 62 for applying same adhesive onto the second sheet member 92. The first applying part 61 is located upstream of the first sheet conveying roller 31 in a conveyance path of the first sheet member 91. In the first applying part 61, the adhesive is applied onto only a plurality of strip-like regions on the first sheet member 91 whose positions are identical to the positions of the plurality of concave portion rows 213 (and the plurality of annular grooves 312) with respect to the axial direction. The second applying part 62 is located upstream of the second sheet conveying roller 41 in a conveyance path of the second sheet member 92. In the second applying part 62, the adhesive is applied onto only a plurality of strip-like regions on the second sheet member 92 whose positions are identical to the positions of the plurality of concave portion rows 213 with respect to the axial direction.

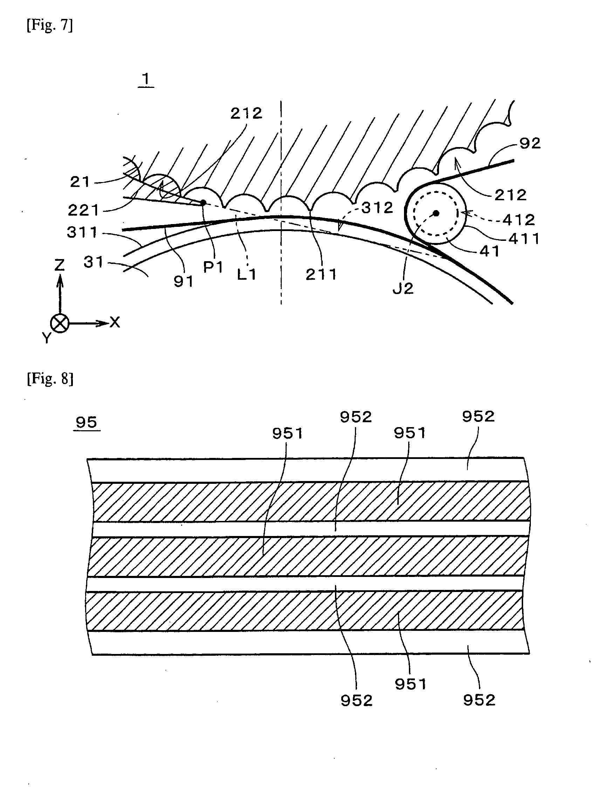

Next, the details of ejection of particles from the cylinder part 21 will be discussed. Fig. 7 is a further enlarged view showing the vicinity of the lowermost portion of the cylinder part 21 in Fig. 4. As shown in Fig. 7, in the cross section of the outer side surface 211 of the cylinder part 21 which is orthogonal to the axial direction, the ejection start position P1 in the vicinity of the lowermost portion of the cylinder part 21 is set at a position lying posterior to (upstream of) the lowermost portion in the rotation direction. As mentioned previously, the cylinder part 21 is rotated at a high speed. Thus, when each of the plurality of concave portions 212 passes the ejection start position P1 (actually, during the passage and immediately after the passage), particles are ejected from the concave portion 212 almost along a tangent line of the outer side surface 211 at the ejection start position P1 (the tangent line is shown in Fig. 7 by a chain double-dashed line denoted by a reference sign L1). In other words, the rotation speed of the cylinder part 21, the shape of the concave portion 212 and the like are determined so that the particles are ejected from the concave portion 212 almost along the tangent line L1 (for example, the particles go toward a direction included in an angle range from (+15) degree to (-15) degree with respect to the tangent line L1 (preferably, from (+10) degree to (-10) degree)). For example, the movement speed of the outer side surface 211 in the direction of the tangent line L1 is 200 meters per minute.

At this time, the tangent line L1 intersects with the outer side surface 311 of the first sheet conveying roller 31, and the positions of the annular grooves 312 of the first sheet conveying roller 31 are identical to the positions of the plurality of concave portion rows 213 with respect to the axial direction (see Fig. 2). Therefore most particles ejected from each concave portion 212 go toward the correspondent annular groove 312. At an intersection point between the tangent line L1 and the outer side surface 311, the first sheet member 91 is in contact with the outer side surface 311.

In the first sheet conveying roller 31, a diameter of the outer side surface 311 is comparatively-large and also the first sheet member 91 is stretched along the outer side surface 311 at a certain tension. Therefore, as shown in Fig. 3, each portion 911 of the first sheet member 91 corresponding to the annular groove 312 becomes a shape depressed toward the bottom of the annular groove 312. In other words, groove portions 911 corresponding to the annular grooves 312 are formed on the first sheet member 91. Thus, the particles ejected from the concave portions 212 of the cylinder part 21 toward the insides of the annular grooves 312 are received (caught) in the groove portions 911. At this time, even if particles bounce from the first sheet member 91 in the groove portions 911, scattering of particles to the outside of the groove portions 911 is suppressed (reduced) by side walls of the groove portions 911. In addition, since adhesive is applied onto the plurality of strip-like regions on the first sheet member 91 whose positions are same as those of the plurality of annular grooves 312 with respect to the axial direction, particles are easily caught in the groove portions 911.

Some particles bounce from the first sheet member 91 in the groove portions 911 to go toward the second sheet conveying roller 41 shown in Fig. 7 (in fact, their flying speeds are decreased by shock absorption by the first sheet member 91), and others go from the concave portions 212 of the cylinder part 21 to the second sheet conveying roller 41 directly. As mentioned previously, in the second sheet conveying roller 41, the plurality of annular grooves 412 are formed whose positions are same as those of the plurality of concave portion rows 213 with respect to the axial direction, and the particles heading to the second sheet conveying roller 41 collide with portions of the second sheet member 92 lying right above the annular grooves 412 (i.e., the portions at which the back surface is not in contact with any substance), so that the impact is absorbed. As a result, some particles are falls into the groove portions 911 of the first sheet member 91 to be collected therein, and others adhere to the surface of the second sheet member 92 where adhesive is applied.

As mentioned previously, immediately after portions on the first sheet member 91 are supplied with particles, the second sheet member 92 is placed on the portions by the second sheet conveying roller 41 in the vicinity of the lowermost portion of the cylinder part 21. Subsequently the first sheet member 91 and the second sheet member 92 are bonded with each other by the bonding roller 51 in Fig. 4 which is a sheet bonding part, to complete the absorbent sheet 95. As above, the second sheet conveying roller 41 and the bonding roller 51, which function as a conveying and bonding part, work harmoniously with the first sheet conveying roller 31, to thereby place and bond the second sheet member 92 on the first sheet member 91.

Fig. 8 is a view showing the absorbent sheet 95 manufactured by the absorbent sheet manufacturing apparatus 1. In the absorbent sheet 95, a plurality of particle existence regions 951 and a plurality of particle non-existence regions 952 are alternately arranged in the width direction. The plurality of particle existence regions 951 are strip-like (or linear) regions where particles of high-absorbent resin are held (in Fig. 8, hatching lines are drawn at the regions), and the plurality of particle non-existence regions 952 are strip-like regions where particles don't exist essentially and the first sheet member 91 and the second sheet member 92 are bonded with each other. In other words, the plurality of particle existence regions 951 are provided in a stripe state in the absorbent sheet 95. In fact, members obtained by cutting the absorbent sheet 95 into same length pieces with respect to the longitudinal direction are used as absorbent parts for absorbent articles. In absorbent articles having such absorbent parts, since adhesive isn't applied on the particle non-existence regions 952, air permeability (breathability) is improved. In order to close both ends of the particle existence regions 951, in each end portion of the member in the longitudinal direction, both sheet members are bonded with each other across the entire length in the width direction, if necessary.

Here, discussion will be made on a comparative example of absorbent sheet manufacturing apparatus. Fig. 9 is a view showing parts of an absorbent sheet manufacturing apparatus 8 in accordance with a comparative example and Fig. 9 corresponds to Fig. 7. In the absorbent sheet manufacturing apparatus 8 of the comparative example, any annular grooves are not formed on the first sheet conveying roller 81 and the second sheet conveying roller 82. In addition, a tangent line (shown in Fig. 9 by a chain double-dashed line denoted by a reference sign 833) of the outer side surface 831 of the cylinder part 83 at the ejection start position 832 doesn't intersect with the outer side surface 811 of the first sheet conveying roller 81. In the absorbent sheet manufacturing apparatus 8 of the comparative example, particles are ejected from the concave portion 834 of the cylinder part 83 almost along the tangent line 833, and some particles go toward the second sheet conveying roller 82. These particles collide with the outer side surface 821 of the second sheet conveying roller 82 through the second sheet member 92 (i.e., collide with the second sheet member 92 held on the second sheet conveying roller 82), to be widely scattered around. Also particles heading to the first sheet conveying roller 81 collide with the outer side surface 811 of the first sheet conveying roller 81 through the first sheet member 91, to be widely scattered around. As above, in the absorbent sheet manufacturing apparatus 8 of the comparative example, scattered particles are wasted and manufacturing cost of the absorbent sheets is increased. In addition, it is difficult to accurately fix particles on regions of the absorbent sheets corresponding to the concave portion rows of the cylinder part 83.

In contrast to this, on the outer side surface 311 of the first sheet conveying roller 31 in the absorbent sheet manufacturing apparatus 1 of Fig. 7, the plurality of annular grooves 312 are formed at positions, respectively, which are identical to the positions of the plurality of concave portion rows 213 of the cylinder part 21 with respect to the axial direction. In addition, the tangent line L1 of the outer side surface 211 at the ejection start position P1 in the cylinder part 21 intersects with the outer side surface 311 of the first sheet conveying roller 31. It is therefore possible to eject particles from the cylinder part 21 into the groove portions 911 of the first sheet member 91 and reduce (suppress) scattering of particles, and as a result, particles can be accurately fixed (settled) on the strip-like regions (particle existence regions 951) of the absorbent sheet 95 corresponding to the annular grooves 312.

The second sheet conveying roller 41 has the annular grooves 412 which extend along substantially the entire length of the outer side surface 411 in the circumferential direction around the second central axis J2 and which face the concave portions 212 of the cylinder part 21. This makes it possible to absorb impact on particles which collide with the second sheet member 92 on the second sheet conveying roller 41, to reduce scattering of particles (the same applies to examples of after-mentioned Figs. 10 to 13). Since waste of particles can be reduced in the absorbent sheet manufacturing apparatus 1, manufacturing cost of the absorbent sheet can be reduced. Furthermore, adhesive is applied onto only the plurality of strip-like regions on the first sheet member 91. This allows particles to be more accurately fixed on the plurality of strip-like regions (particle existence regions 951) of the absorbent sheet 95.

Fig. 10 is a view showing another example of absorbent sheet manufacturing apparatus and Fig. 10 corresponds to Fig. 4. In an absorbent sheet manufacturing apparatus 1 in accordance with another example, with respect to a moving direction (the X direction in Fig. 10) of the outer side surface 211 of the cylinder part 21 at the lowermost portion, the first central axis J1 of the first sheet conveying roller 31 lies anterior to (on the (+X) side of) the rotation axis R1 of the cylinder part 21. In fact, the position of the particle supplying part 2 shown in Fig. 1 is slightly moved leftward and downward (toward the (-X) side and (-Z) side) in Fig. 1 relative to the other members, and then the position of the second sheet conveying roller 41 is adjusted so that the relative position of the second central axis J2 of the second sheet conveying roller 41 with respect to the rotation axis R1 of the cylinder part 21 and the first central axis J1 of the first sheet conveying roller 31 becomes same as that in Fig. 1. Therefore, the cylinder part 21, the first sheet conveying roller 31 and the second sheet conveying roller 41 are located at positions shown in Fig. 10. In the absorbent sheet manufacturing apparatus 1 in Fig. 10, a state where the particle filling opening 232 (see Fig. 1) is parallel to the horizontal plane is maintained. In addition, if necessary, the positions of the plurality of auxiliary rollers 32 (see Fig. 1) are adjusted so that a line segment L2 (shown in Fig. 10 by a chain double-dashed line) connecting the rotation axis R1 of the cylinder part 21 and the first central axis J1 of the first sheet conveying roller 31 becomes almost orthogonal to the first sheet member 91.

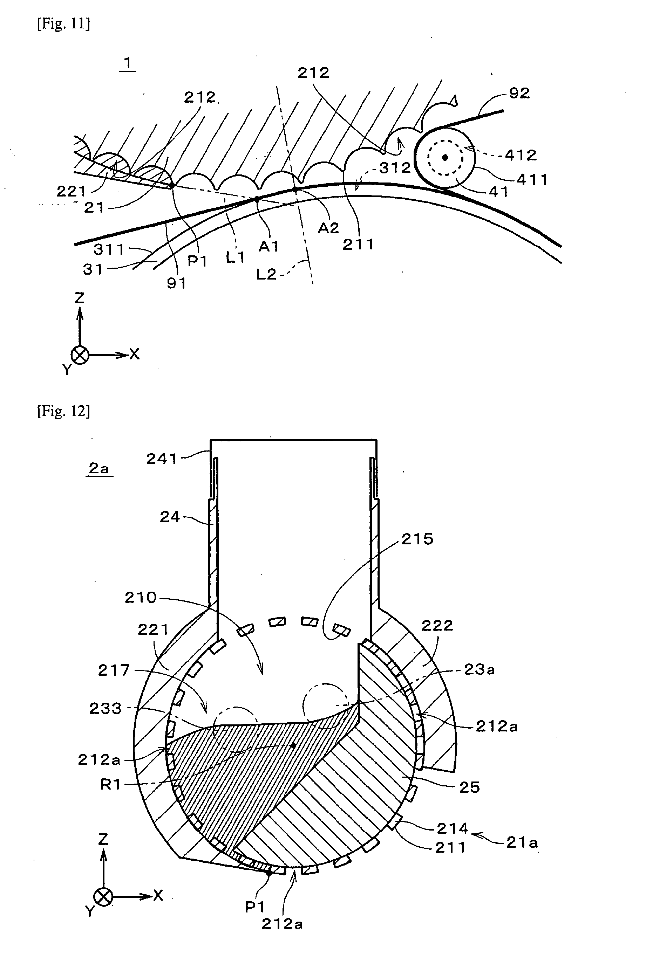

Fig. 11 is an enlarged view showing the vicinity of the lowermost portion of the cylinder part 21 in Fig. 10 and Fig. 11 corresponds to Fig. 7. As shown in Fig. 11, in an absorbent sheet manufacturing apparatus 1 in accordance with another example, the position of the first cover part 221 is adjusted so that a position at which the tangent line L1 of the outer side surface 211 at the ejection start position P1 in the cylinder part 21 intersects with a bottom surface of an annular groove 312 of the first sheet conveying roller 31 lies on the (-X) side of the uppermost portion of the first sheet conveying roller 31. Thus, particles directly heading toward the second sheet conveying roller 41 from the cylinder part 21 are decreased in particles ejected from the cylinder part 21.

In addition, in the absorbent sheet manufacturing apparatus 1 of Fig. 11, a first intersection point A1 at which the above tangent line L1 intersects with the outer side surface 311 of the first sheet conveying roller 31 lies posterior to (upstream of) a second intersection point A2 in the rotation direction of the first sheet conveying roller 31, at which the above line segment L2 intersects with the outer side surface 311 of the first sheet conveying roller 31. Thus, particles are ejected toward the vicinity of the posterior side, in the rotation direction of the first sheet conveying roller 31, of the portion (the portion in the vicinity of the second intersection point A2) which is nearest to the outer side surface 211 of the cylinder part 21, in the outer side surface 311 of the first sheet conveying roller 31. Therefore, even if particles bounce from the first sheet member 91 in the annular grooves 312, scattering of particles can be suppressed by portions of the outer side surface 211 of the cylinder part 21 which are near (close to) the first sheet conveying roller 31, and particles can be more accurately fixed on the strip-like regions of the absorbent sheet 95 corresponding to the annular grooves 312.

In the absorbent sheet manufacturing apparatus 1 of Fig. 4, by rotating the first cover part 221 posteriorly (clockwise) in the rotation direction of the cylinder part 21 around the rotation axis R1 by a predetermined angle, the first intersection point at which the tangent line of the outer side surface 211 at the ejection start position (the tip of the first cover part 221) intersects with the outer side surface 311 of the first sheet conveying roller 31 can lie posterior to the second intersection point in the rotation direction of the first sheet conveying roller 31, at which the line segment connecting the rotation axis R1 and the first central axis J1 intersects with the outer side surface 311. However, it is preferable that the position of the first central axis J1 of the first sheet conveying roller 31 and the position of the rotation axis R1 of the cylinder part 21 are displaced in the X direction like the absorbent sheet manufacturing apparatus 1 in Fig. 10, in order to eject particles almost along a horizontal direction at the ejection start position (for example, if an angle formed between the tangent line of the outer side surface 211 at the ejection start position and the X direction is made equal to or less than 30 degree).

Although particles are ejected from the concave portions 212 on the outer side surface 211 toward the first sheet member 91 in the particle supplying part 2 of Fig. 1, another particle supplying part may be utilized in the absorbent sheet manufacturing apparatus 1. Fig. 12 is a view showing another example of particle supplying part.

In the particle supplying part 2a of Fig. 12, the cylinder part 21a is a generally cylindrical member around the rotation axis R1 and has a ring-like side wall 214. A cylindrical exhaust part 24 is provided above the cylinder part 21a as substitute for the particle filling part 23 in Fig. 1, and a pouched filter 241 formed of nonwoven fabric or the like is attached to an upper portion of the exhaust part 24. The first cover part 221 and the second cover part 222 similar to those in Fig. 1 are provided around the cylinder part 21a.

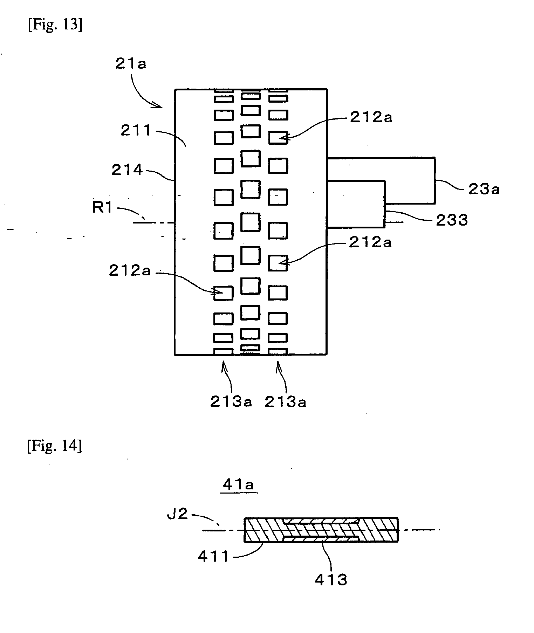

Fig. 13 is a view showing an outer side surface 211 of the side wall 214 of the cylinder part 21a, and in Fig. 13, an appearance of the outer side surface 211 which is observed along a direction orthogonal to the rotation axis R1 is shown. In Fig. 13, the first and second cover parts 221, 222 are omitted. As shown in Figs. 12 and 13, the cylinder part 21 has a plurality of through-holes 212a which are holes passing through the side wall 214. The plurality of through-holes 212a are arranged in the circumferential direction around the rotation axis R1 with respect to each of a plurality of positions in the axial direction. When the plurality of through-holes 212a which are arranged in the circumferential direction at the same position in the axial direction are referred to as a through-hole row 213a, three through-hole rows 213a are provided in the cylinder part 21a shown in Fig. 13.

As shown in Fig. 12, an isolation part 25 which covers a portion of an inner side surface 215 of the side wall 214 of the cylinder part 21a is provided in the internal space 210 of the cylinder part 21a. The isolation part 25 is provided in the right portion of the internal space 210 in Fig. 12 and covers the right portion of the inner side surface 215 from the vicinity of the lowermost portion of the cylinder part 21a to the vicinity of the uppermost portion. The isolation part 25 faces a portion of the side wall 214 positioned from the lower end portion of the first cover part 221 to the upper end portion of the second cover part 222 with respect to the rotation direction of the cylinder part 21a (in the counterclockwise direction in Fig. 12). In the cylinder part 21a, a portion of the internal space 210 where the isolation part 25 doesn't exist is a particle storage space 217 which stores particles of high-absorbent resin. The isolation part 25 is provided across almost the entire width of the inner side surface 215 of the cylinder part 21a in the axial direction, so that through-holes 212a in a region of the inner side surface 215 which is covered with the isolation part 25 are isolated from the particle storage space 217. Thus, also in a region of the inner side surface 215 which is from the tip of the first cover part 221 (i.e., ejection start position P1) to the lower end portion of the second cover part 222, through-holes 212a are isolated from the particle storage space 217.

The particle supplying part 2a has a particle replenishment part 23a (in Fig. 12, the particle replenishment part 23a is shown by a chain double-dashed line, and the same applies to an after-mentioned level sensor 233) provided to the right end portion of the cylinder part 21a in Fig. 13. The particle replenishment part 23a is a screw feeder which has a screw therein, and replenishes particles into the particle storage space 217 of the cylinder part 21a from one end portion (the right end portion in Fig. 13) of the cylinder part 21a in the axial direction. A level sensor 233 is provided in the particle storage space 217. When the amount of particles stored in the particle storage space 217 becomes equal to or less than a certain level, replenishment of particles is performed. When particles are replenished into the particle storage space 217, air in the particle storage space 217 is exhausted mainly through the exhaust part 24. Even if particles go out into the exhaust part 24 from the cylinder part 21a, the particles are prevented by the filter 241 from going outside of the absorbent sheet manufacturing apparatus 1.

In the absorbent sheet manufacturing apparatus 1, the cylinder part 21a in Fig. 12 is rotated at a high speed around the rotation axis R1, so that particles in the particle storage space 217 are filled into through-holes 212a facing particles stored in the particle storage space 217 in the cylinder part 21a, out of the plurality of through-holes 212a. Until each through-hole 212a filled with particles reaches the ejection start position P1, the outer end of the through-hole 212a is closed (blocked) with the first cover part 221. The through-hole 212a is moved to a position opposed to the isolation part 25, and therefore the particles in the through-hole 212a is isolated (separated) from particles in the particle storage space 217. Then, when each through-hole 212a filled with particles passes the ejection start position P1 (i.e., the tip of the first cover part 221), the particles in the through-hole 212a are ejected toward the first sheet member 91.

Also in the absorbent sheet manufacturing apparatus 1 having the particle supplying part 2a in Fig. 12, the tangent line of the outer side surface 211 of the cylinder part 21a at the ejection start position P1 intersects with the outer side surface 311 of the first sheet conveying roller 31. In addition, the plurality of annular grooves 312 are formed on the outer side surface 311 of the first sheet conveying roller 31 at the same positions as those of the plurality of through-hole rows 213a of the cylinder part 21a with respect to the axial direction. Therefore, scattering of particles is reduced, and as a result, particles can be accurately fixed on the strip-like regions of the absorbent sheet 95 corresponding to the annular grooves 312.

Though the preferred embodiments of the present invention have been discussed above, the present invention is not limited to the above-discussed preferred embodiments, but allows various variations.

In the second sheet conveying roller 41, the plurality of annular grooves 412 corresponding to the plurality of concave portion rows 213 of the cylinder part 21 (or the plurality of through-hole rows 213a of the cylinder part 21a) are not necessarily formed, and for example, one annular groove having a width across the entire extent corresponding to all concave portion rows 213 with respect to the axial direction may be formed (the width of the annular groove is less than the width of the second sheet member 92). In this case, impact on particles which collide with the second sheet member 92 on the second sheet conveying roller 41 can be absorbed by a gap formed between the bottom surface of the annular groove and the second sheet member 92. If a groove extending along substantially the entire length of the outer side surface in the circumferential direction around the second central axis J2 is formed so as to face holes of the cylinder part, the groove isn't necessarily annular and the groove may be a spiral groove along the second central axis J2 on the outer side surface 411, for example.

From the viewpoint of absorbing impact on particles which collide with the second sheet member 92 on the second sheet conveying roller 41, as shown in Fig. 14, a second sheet conveying roller 41a having an absorber (shock absorber) 413 on the outer side surface 411 which is a sponge, elastic rubber or the like configured to absorb impact on particles which collide with the second sheet member 92 thereon may be employed. The absorber 413 in Fig. 14 is annular around the second central axis J2 and is provided across the entire extent corresponding to all concave portion rows 213 of the cylinder part 21 with respect to the axial direction.

Depending on the design of the particle supplying part, for example, the first cover part 221 is omitted in the particle supplying part 2 of Fig. 1, and a suction mechanism for suctioning particles in each concave portion 212 of the cylinder part 21 to hold the particles therein may be provided additionally. In this case, the ejection start position is set as a position to release the suction in the suction mechanism.

In the absorbent sheet manufacturing apparatus 1, the groove portions 911 of the first sheet member 91 can reduce scattering of particles from the first sheet member 91. Thus, even if not applying adhesive onto the first sheet member 91, particles can be fixed on the plurality of strip-like regions of the absorbent sheet with some accuracy. Depending on the design of the absorbent sheet, adhesive may be applied over the entire surface of the first sheet member 91.

In the bonding roller 51, there may be a case where an annular groove similar to that of the first sheet conveying roller 31 is formed, to avoid contact of both the first sheet conveying roller 31 and the bonding roller 51 with the strip-like regions (regions of the groove portions 911) on which particles are fixed in bonding between the first sheet member 91 and the second sheet member 92.

Depending on the design of the absorbent sheet manufacturing apparatus 1, there may be a case where the first sheet member 91 and the second sheet member 92 are bonded with each other between the second sheet conveying roller 41 and the first sheet conveying roller 31 and the bonding roller 51 is omitted. In this case, only the second sheet conveying roller 41 realizes the conveying and bonding part for placing the second sheet member 92 on the first sheet member 91 to bond the second sheet member 92 on the first sheet member 91.

In the above preferred embodiment, discussion has been made on the manufacture (production) of the absorbent sheets 95 where the striped particle existence regions 951 are set, however, the technique where the groove portions 911 of the first sheet member 91 reduce scattering of particles may be utilized for the manufacture of absorbent sheets 95 each having only one particle existence region 951. In this case, the first sheet conveying roller 31 may be provided with only one annular groove.

In the above absorbent sheet manufacturing apparatus, particles of absorbent material are supplied such as crosslinked partially neutralized polyacrylic acid, hydrolyzed starch-acrylic acid graft polymer, saponified vinyl acetate-acrylic ester copolymer, hydrolyzed acrylonitrile copolymer, crosslinked acrylonitrile copolymer, hydrolyzed acrylamide copolymer, crosslinked acrylamide copolymer, crosslinked cationic monomers, or crosslinked polyamino acid.

Structure of the absorbent sheet manufacturing apparatus may be utilized for a sheet article manufacturing apparatus for manufacturing a deodorant sheet which is a sheet article for an absorbent article such as a disposable diaper or absorbent pad for light incontinence, by supplying particles of deodorant material such as activated carbon, silica, alumina, zeolite, ion-exchange resin, or molecular sieve onto the first sheet member 91.

The constituent elements of above-discussed preferred embodiments and modified examples may be appropriately combined with one another, as long as they are not mutually exclusive.

While the invention has been shown and described in detail, the foregoing description is in all aspects illustrative and not restrictive. It is therefore understood that numerous modifications and variations can be devised without departing from the scope of the invention.

### Reference Signs List

1 Absorbent sheet manufacturing apparatus
21, 21a Cylinder part
31 First sheet conveying roller
41, 41a Second sheet conveying roller
51 Bonding roller
61 First applying part
91 First sheet member
92 Second sheet member
95 Absorbent sheet
211, 311, 411 Outer side surface
212 Concave portion
212a Through-hole
312, 412 Annular groove
413 Absorber
951 Particle existence region
A1, A2 Intersection point
J1, J2 Central axis
L1 Tangent line
L2 Line segment
P1 Ejection start position
R1 Rotation axis

## Claims

1. A sheet article manufacturing apparatus (1) for manufacturing a sheet article (95) for an absorbent article, comprising:
a cylinder part (21, 21 a) having an outer side surface (211) which is generally cylindrical around a rotation axis (R1) along a horizontal direction, a plurality of holes (212, 212a) each filled with particles of absorbent material or deodorant material being arranged on said outer side surface in a circumferential direction around said rotation axis, said outer side surface being rotated around said rotation axis in a predetermined rotation direction, said cylinder part ejecting said particles almost along a tangent line (L1) of said outer side surface at an ejection start position (P1) when each of said plurality of holes passes said ejection start position, said ejection start position being set at a position in a vicinity of a lowermost portion in a cross section of said outer side surface which is orthogonal to said rotation axis, said position lying posterior to said lowermost portion in said rotation direction;
a sheet conveying roller (31) which is located near said lowermost portion of said cylinder part, said sheet conveying roller having an outer side surface (311) which is generally cylindrical around a central axis (J1) parallel to said rotation axis, said outer side surface being rotated around said central axis in a rotation direction opposite to said rotation direction of said cylinder part to convey a first sheet member (91) along said outer side surface which is continuous sheet and cause said first sheet member to pass in the vicinity of said lowermost portion of said cylinder part; and
a conveying and bonding part (41, 41a, 51) for placing, in the vicinity of said lowermost portion of said cylinder part, a second sheet member (92) on said first sheet member which has been supplied with said particles to bond said second sheet member on said first sheet member, said second sheet member being continuous sheet; and **characterized in that**
said tangent line intersects with said outer side surface of said sheet conveying roller, and
an annular groove (312) along a circumferential direction around said central axis is formed on said outer side surface of said sheet conveying roller so as to face holes of said cylinder part.

2. The sheet article manufacturing apparatus according to claim 1, wherein
a first intersection point (A1) at which said tangent line intersects with said outer side surface of said sheet conveying roller lies posterior to a second intersection point (A2) in said rotation direction of said sheet conveying roller, at which a line segment (L2) connecting said rotation axis of said cylinder part and said central axis of said sheet conveying roller intersects with said outer side surface of said sheet conveying roller.

3. The sheet article manufacturing apparatus according to claim 2, wherein
with respect to a moving direction of said outer side surface of said cylinder part at said lowermost portion,
said central axis of said sheet conveying roller lies anterior to said rotation axis of said cylinder part.

4. The sheet article manufacturing apparatus according to any one of claims 1 to 3, wherein
said conveying and bonding part comprises:
another sheet conveying roller (41, 41a) which is located anterior to said lowermost portion of said cylinder part with respect to a moving direction of said outer side surface at said lowermost portion, said another sheet conveying roller having an outer side surface (411) which is generally cylindrical around a central axis (J2) parallel to said rotation axis, for conveying said second sheet member along said outer side surface to the vicinity of said lowermost portion to place said second sheet member on said first sheet member which has been supplied with said particles; and
a sheet bonding part (51) for bonding said second sheet member on said first sheet member; wherein said another sheet conveying roller has a groove (412) or an absorber (413) on said outer side surface,
said groove extending along substantially the entire length of said outer side surface in a circumferential direction around said central axis and facing holes of said cylinder part, said absorber being configured to absorb impact on said particles which collide with said second sheet member on said outer side surface.

5. The sheet article manufacturing apparatus according to any one of claims 1 to 4, wherein
said plurality of holes are formed on said outer side surface of said cylinder part with respect to each of a plurality of positions in an axial direction parallel to said rotation axis, and
said annular groove is formed on said outer side surface of said sheet conveying roller with respect to each of said plurality of positions in said axial direction.

6. The sheet article manufacturing apparatus according to claim 5, further comprising an applying part (61) for applying adhesive onto a plurality of strip-like regions (951) lying on said first sheet member, said plurality of strip-like regions corresponding to said plurality of positions in said axial direction.

## Patentansprüche

1. Folienartikelherstellungsvorrichtung (1) zum Herstellen eines Folienartikels (95) für einen Absorptionsartikel, mit
einem Zylinderbauteil (21, 21a), das eine Außenfläche (211) hat, die im Allgemeinen zylindrisch um eine Drehachse (R1) entlang einer horizontalen Richtung ist,
einer Vielzahl von Löchern (212, 212a), wobei jedes mit Partikeln eines Absorptionsmittels oder Desodoransmittels gefüllt an der Außenfläche in einer Umfangsrichtung um die Drehachse angeordnet ist, wobei, wenn die Außenfläche um die Drehachse in einer vorgegebenen Drehrichtung dreht, das Zylinderbauteil die Partikel annähernd entlang einer Tangente (L1) der Außenfläche an einer Abwurfstartposition (P1) auswirft, wenn jedes der Vielzahl von Löchern die Abwurfstartposition passiert, wobei die Abwurfstartposition an einer Position in einer Nähe eines tiefsten Abschnitts in einem Querschnitt der Außenfläche festgelegt ist, der senkrecht zu der Drehachse ist, wobei die Position hinter dem tiefsten Abschnitt in der Drehrichtung liegt;
einer Folienförderwalze (31), die nahe dem tiefsten Abschnitt des Zylinderbauteils liegt, wobei die Folienförderwalze eine Außenfläche (311) hat, die im Allgemeinen zylindrisch um eine Mittelachse (J1) ist, die parallel zu der Drehachse ist, wobei die Außenfläche um die Mittelachse in einer Drehrichtung drehbar ist, die der Drehrichtung des Zylinderbauteils entgegengesetzt ist, um ein erstes Folienelement (91), das eine Endlosfolie ist, entlang der Außenfläche zu fördern und zu bewirken, dass das erste Folienelement in der Nähe des tiefsten Abschnitts des Zylinderbauteils vorbeikommt; sowie
einem Förder- und Kaschierbauteil (41, 41a, 51) zum Positionieren eines zweiten Folienelements (92), in der Nähe des tiefsten Abschnitts des Zylinderbauteils, auf dem ersten Folienelement, dem die Partikel zugeführt wurden, um das zweite Folienelement auf das erste Folienelement zu kaschieren, wobei das zweite Folienelement eine Endlosfolie ist; und **dadurch gekennzeichnet, dass**
die Tangente die Außenfläche der Folienförderwalze schneidet und eine Ringnut (312) entlang einer Umfangsrichtung um die Mittelachse an der Außenfläche der Folienförderwalze, den Löchern des Zylinderbauteils gegenüberliegend, ausgebildet ist.

2. Folienartikelherstellungsvorrichtung nach Anspruch 1, wobei
ein erster Schnittpunkt (A1), an dem die Tangente die Außenfläche der Folienförderwalze schneidet, hinter einem zweiten Schnittpunkt (A2) in der Drehrichtung der Folienförderwalze liegt, an dem eine Strecke (L2), die die Drehachse des Zylinderbauteils und die Mittelachse der Folienförderwalze verbindet, die Außenfläche der Folienförderwalze schneidet.

3. Folienartikelherstellungsvorrichtung nach Anspruch 2, wobei,
bezogen auf eine Bewegungsrichtung der Außenfläche des Zylinderbauteils an dem tiefsten Abschnitt, die Mittelachse der Folienförderwalze vor der Drehachse des Zylinderbauteils liegt.

4. Folienartikelherstellungsvorrichtung nach einem der Ansprüche 1 bis 3, wobei das Förder- und Kaschierbauteil folgendes umfasst:
eine weitere Folienförderwalze (41, 41a), die, bezogen auf eine Bewegungsrichtung der Außenfläche an dem tiefsten Abschnitt, vor dem tiefsten Abschnitt des Zylinderbauteils liegt, wobei die weitere Folienförderwalze eine Außenfläche (411) hat, die im Allgemeinen zylindrisch um eine Mittelachse (J2) ist, die parallel zu der Drehachse ist, um das zweite Folienelement entlang der Außenfläche in die Nähe des tiefsten Abschnitts zu fördern, um das zweite Folienelement auf dem ersten Folienelement, dem die Partikel zugeführt wurden, zu positionieren; sowie
ein Folienkaschierbauteil (51), zum Kaschieren des zweiten Folienelements auf dem ersten Folienelement; wobei
die weitere Folienförderwalze eine Nut (412) oder ein Dämpfungselement (413) auf der Außenfläche hat, wobei sich die Nut entlang im Wesentlichen der gesamten Länge der Außenfläche in einer Umfangsrichtung um die Mittelachse erstreckt und den Löchern des Zylinderbauteils gegenüberliegt, wobei das Dämpfungselement eingerichtet ist, um Stöße auf die Partikel zu dämpfen, die mit dem zweiten Folienelement auf der Außenfläche zusammenstoßen.

5. Folienartikelherstellungsvorrichtung nach einem der Ansprüche 1 bis 4, wobei
die Vielzahl von Löchern auf der Außenfläche des Zylinderbauteils ausgebildet ist, bezogen auf jede einer Vielzahl von Positionen in einer Axialrichtung, die parallel zu der Drehrichtung ist, und
die Ringnut auf der Außenfläche der Folienförderwalze ausgebildet ist, bezogen auf jede der Vielzahl von Positionen in der Axialrichtung.

6. Folienartikelherstellungsvorrichtung nach Anspruch 5, ferner mit
einem Auftragebauteil (61), zum Auftragen von Klebstoff auf eine Vielzahl von streifenförmigen Bereichen (951), die auf dem ersten Folienelement gelegen sind, wobei die Vielzahl von streifenförmigen Bereichen der Vielzahl von Positionen in der Axialrichtung zugeordnet ist.

## Revendications

1. Appareil de fabrication d'article de feuille (1) pour fabriquer un article de feuille (95) pour un article absorbant, comprenant :
une partie de cylindre (21, 21a) ayant une surface latérale externe (211) qui est globalement cylindrique autour d'un axe de rotation (R1) le long d'une direction horizontale, une pluralité de trous (212, 212a), chacun rempli de particules de matériau absorbant ou de matériau désodorisant étant agencés sur ladite surface latérale externe dans une direction circonférentielle autour dudit axe de rotation, ladite surface latérale externe étant entraînée en rotation autour dudit axe de rotation dans une direction de rotation prédéterminée, ladite partie de cylindre éjectant lesdites particules presque le long d'une tangente (L1) de ladite surface latérale externe à une position de début d'éjection (P1) lorsque chacun de ladite pluralité de trous passe à ladite position de début d'éjection, ladite position de début d'éjection étant fixée à une position à proximité de la partie la plus basse en coupe transversale de ladite surface latérale externe qui est orthogonale audit axe de rotation, ladite position se trouvant à l'arrière de ladite partie la plus basse dans ladite direction de rotation ;
un rouleau de transport de feuilles (31) qui est situé à côté de ladite partie la plus basse de ladite partie de cylindre, ledit rouleau de transport de feuilles ayant une surface latérale externe (311) qui est globalement cylindrique autour d'un axe central (J1) parallèle audit axe de rotation, ladite surface latérale externe étant entraînée en rotation autour dudit axe central dans une direction de rotation opposée à ladite direction de rotation de ladite partie de cylindre pour transporter un premier élément de feuille (91) le long de ladite surface latérale externe qui est une feuille continue et pour amener ledit premier élément de feuille à passer à proximité de ladite partie la plus basse de ladite partie de cylindre ; et
une partie de transport et de fixation (41, 41a, 51) pour placer, à proximité de ladite partie la plus basse de ladite partie de cylindre, un deuxième élément de feuille (92) sur ledit premier élément de feuille qui a été alimenté en lesdites particules pour fixer ledit deuxième élément de feuille sur ledit premier élément de feuille, ledit deuxième élément de feuille étant une feuille continue ; et **caractérisé en ce que**
ladite tangente coupe ladite surface latérale externe dudit rouleau de transport de feuilles, et
une rainure annulaire (312) le long d'une direction circonférentielle autour dudit axe central est formée sur ladite surface latérale externe dudit rouleau de transport de feuilles afin de faire face à des trous de ladite partie de cylindre.

2. Appareil de fabrication d'article de feuille selon la revendication 1, dans lequel
un premier point d'intersection (A1) au niveau duquel ladite tangente coupe ladite surface latérale externe dudit rouleau de transport de feuilles se trouve à l'arrière d'un deuxième point d'intersection (A2) dans ladite direction de rotation dudit rouleau de transport de feuilles, au niveau duquel un segment de droite (L2) reliant ledit axe de rotation de ladite partie de cylindre et ledit axe central dudit rouleau de transport de feuilles coupe ladite surface latérale externe dudit rouleau de transport de feuilles.

3. Appareil de fabrication d'article de feuille selon la revendication 2, dans lequel
par rapport à une direction de déplacement de ladite surface latérale externe de ladite partie de cylindre au niveau de ladite partie la plus basse, ledit axe central dudit rouleau de transport de feuilles se trouve à l'avant dudit axe de rotation de ladite partie de cylindre.

4. Appareil de fabrication d'article de feuille selon l'une quelconque des revendications 1 à 3, dans lequel
ladite partie de transport et de fixation comprend :
un autre rouleau de transport de feuilles (41, 41a) qui est situé à l'avant de ladite partie la plus basse de ladite partie de cylindre par rapport à une direction de déplacement de ladite surface latérale externe au niveau de ladite partie la plus basse, ledit autre rouleau de transport de feuilles ayant une surface latérale externe (411) qui est globalement cylindrique autour d'un axe central (J2) parallèle audit axe de rotation, pour transporter ledit deuxième élément de feuille le long de ladite surface latérale externe à proximité de ladite partie la plus basse pour placer ledit deuxième élément de feuille sur ledit premier élément de feuille qui a été alimenté en lesdites particules ; et
une partie de fixation de feuilles (51) pour fixer ledit deuxième élément de feuille sur ledit premier élément de feuille ; dans lequel
ledit autre rouleau de transport de feuilles a une rainure (412) ou un amortisseur (413) sur ladite surface latérale externe, ladite rainure s'étendant substantiellement le long de toute la longueur de ladite surface latérale externe dans une direction circonférentielle autour dudit axe central et faisant face à des trous de ladite partie de cylindre, ledit amortisseur étant configuré pour amortir le choc sur lesdites particules qui entrent en collision avec ledit deuxième élément de feuille sur ladite surface latérale externe.

5. Appareil de fabrication d'article de feuille selon l'une quelconque des revendications 1 à 4, dans lequel
ladite pluralité de trous sont formés sur ladite surface latérale externe de ladite partie de cylindre par rapport à chacune d'une pluralité de positions dans une direction axiale parallèle audit axe de rotation, et
ladite rainure annulaire est formée sur ladite surface latérale externe dudit rouleau de transport de feuilles par rapport à chacune de ladite pluralité de positions dans ladite direction axiale.

6. Appareil de fabrication d'article de feuille selon la revendication 5, comprenant en outre
une partie d'application (61) pour appliquer de l'adhésif sur une pluralité de régions en forme de bande (951) se trouvant sur ledit premier élément de feuille, ladite pluralité de régions en forme de bande correspondant à ladite pluralité de positions dans ladite direction axiale.
